# EUROPÄISCHE PATENTSCHRIFT

(11) **EP 2 814 407 B1**
(45) Veröffentlichungstag und Bekanntmachung des Hinweises auf die Patenterteilung: **30.11.2016**
(21) Anmeldenummer: 13703362.7
(22) Anmeldetag: 31.01.2013
(51) Int. Cl.: A61B 17/16, A61B 17/295, A61B 17/00, A61B 90/00

(54) **ZERLEGBARES CHIRURGISCHES SCHIEBESCHAFTINSTRUMENT**
SURGICAL SLIDING SHAFT INSTRUMENT THAT CAN BE DISMANTLED
INSTRUMENT CHIRURGICAL À TIGE COULISSANTE DÉMONTABLE

(30) Priorität: 13.02.2012 DE 202012001348 U
(43) Veröffentlichungstag der Anmeldung: 24.12.2014
(73) Patentinhaber: Waldemar Link GmbH & Co. KG, 22339 Hamburg (DE)
(72) Erfinder: DMUSCHEWSKY, Klaus, 22397 Hamburg (DE)
(74) Vertreter: Glawe, Delfs, Moll
(86) Internationale Anmeldenummer: PCT/EP2013/051941
(87) Internationale Veröffentlichungsnummer: WO 2013/120701

(56) Entgegenhaltungen:
- EP-A1- 1 491 155
- EP-A2- 0 706 780
- EP-A2- 1 212 983
- DE-A1-102008 034 287
- DE-C2- 19 748 369
- DE-U1- 20 103 630
- US-A1- 2003 088 268

## Beschreibung

Die Erfindung betrifft den Verriegelungsmechanismus eines zerlegbaren chirurgischen Schiebeschaftinstruments.

Chirurgische Instrumente, insbesondere chirurgische Schiebeschaftinstrumente müssen nach jedem Gebrauch gereinigt werden. Dabei ist es nötig, auch Flächen zu reinigen, die zwischen verschiedenen Komponenten der Instrumente angeordnet sind. Dazu werden die Instrumente in mehrere Teile zerlegbar ausgeführt. Das Instrument weist an einem Führungselement eine entlang eines Schieberelements ausgerichtete Schiene mit einem proximalen und distalen Teil auf. Der distale Teil der Schiene weist ein hinterschnittenes Profil auf. Der proximale Teil der Schiene weist eine Montageöffnung auf. Weiter weist das Schiebeschaftinstrument ein Schieberelement auf, das einen Profilsteg umfasst, der ein zum Schienenprofil komplementäres Profil aufweist. Der Profilsteg greift dabei in die Schiene ein. Das Schieberelement, welches gleitend auf einem Führungselement angeordnet ist, kann durch die Zerlegung von dem Führungselement gelöst werden, so dass die Gleitflächen gereinigt werden können. Dabei wird das Schieberelement mit dem Profilsteg zum proximalen Ende der Schiene geschoben, so dass der Profilsteg in der Montageöffnung angeordnet ist, aus der er aus der Schiene gelöst werden kann. Um eine Zerlegung während eines chirurgischen Eingriffs zu verhindern, ist ein Verriegelungsmechanismus vorgesehen.

Aus der DE 20 2008 001 675 U1 ist bekannt, die Zerlegung des Schiebeschaftinstruments durch weites Öffnen zweier Griffteile einzuleiten. Ein Schieberelement, das gleitend auf einem Führungselement gelagert ist, weist dazu an seinem proximalen Ende eine Führungsnut auf, die aus zwei Teilen besteht. Die Führungsnut ist in der Bewegungsrichtung des Schieberelements ausgerichtet. Der distale Teil der Führungsnut ist schmaler als der proximale Teil der Führungsnut ausgeführt. Weiter weist das Führungselement an seinem proximalen Ende einen Verriegelungsschieber auf, der in den proximalen Teil der Führungsnut eingreift. Der Übergang zwischen dem proximalen und dem distalen Teil der Führungsnut ist durch Radien verrundet ausgestaltet. Mittels des Übergangs wird das Schieberelement beim Öffnen der Griffe des Schiebeschaftinstruments durch den Verriegelungsschieber gestoppt, so dass eine Zerlegung des Schiebeschaftinstruments verhindert wird.

Die DE 197 48 369 C2 betrifft ein zerlegbares Schiebeschaftinstrument, das als Verriegelungsmechanismus eine Drehsicherung aufweist. Die Drehsicherung ist als drehbar gelagerte Scheibe ausgestaltet. Die Scheibe ist so am proximalen Ende des Führungselements angeordnet, dass sie den Weg des Schieberelements blockiert. Die Scheibe weist eine Ausnehmung auf, die zur Entriegelung zum Schieberelement gedreht wird. Dadurch wird die Blockade des Schieberelements aufgehoben. Weiter kann das Verriegelungselement auch am beweglichen Griffteil des Schiebeschaftinstruments angeordnet werden. Dabei blockiert der Verriegelungsmechanismus die Öffnung des zweiten Griffteils durch einen Formschluss mit der Unterseite des Führungselements.

Die oben aufgeführten Verriegelungsmechanismen weisen den gemeinsamen Nachteil auf, dass sie keine sichere Verriegelung gewährleisten können. So kann bei der als Schieber ausgeführten Form des Verriegelungsmechanismus durch die mit Radien verrundet ausgestaltete Führungsnut ein Verbiegen des Verriegelungsschiebers herbeigeführt werden. Weiter kann der Verriegelungsschieber durch ein kräftiges Öffnen der Griffelemente des Schiebeschaftinstruments verbogen werden, so dass in beiden Fällen eine unbeabsichtigte Zerlegung des Schiebeschaftinstruments stattfinden kann. In der Ausführungsform mit der Drehsicherung kann durch die freie Drehbarkeit der Sicherung ein versehentliches Öffnen herbeigeführt werden. So kann auch in dieser Ausführungsform eine versehentliche Zerlegung während eines chirurgischen Eingriffs stattfinden.

Weiter ist bekannt die Drehsicherung mit einem Verriegelungselement auszustatten. Das Verriegelungselement ist dabei auf einem Achsstumpf angeordnet, der mit einer am Griffteil angeordneten Buchse gekoppelt ist. Der Achsstumpf ist weiter in seiner axialen Richtung beweglich gelagert. Die Buchse weist weiter eine Ausnehmung auf, in die das Verriegelungselement bei Sicherung des Schieberelements angeordnet wird. Nachteilig an dieser Vorrichtung ist, dass die Drehsicherung in verschiedenen Positionen eine Verriegelung des Schieberelements herbeiführen kann. Allerdings ist nur in einer Position, in der das Verriegelungselement in der Ausnehmung der Buchse angeordnet ist, eine sichere Verriegelung gewährleistet. Daher kann es bei einer falschen Positionierung der Drehsicherung zu einer unbeabsichtigten Zerlegung des Schiebeschaftinstruments kommen.

Die Aufgabe der Erfindung ist damit ein zu weites Öffnen des zerlegbaren Schiebeschaftinstruments zu verhindern und dabei eine sichere Verriegelung des Zerlegmechanismus zu gewährleisten.

Die Aufgabe wird gelöst durch die Gegenstände der unabhängigen Ansprüche. Vorteilhafte Weiterbildungen sind Gegenstand der abhängigen Ansprüche.

Eine erste Alternative der Erfindung umfasst ein zerlegbares chirurgisches Schiebeschaftinstrument umfassend einen Funktionsschaft, der ein Führungselement und ein Schieberelement, das an dem Führungselement gleitend gelagert ist und ein Kopplungselement aufweist, sowie eine entlang der Schieberichtung des Schieberelements ausgerichtete hinterschnittene Schiene, die eine Montageöffnung aufweist, und einen Profilsteg mit einem zur Schiene komplementären Profil umfasst, ein Griffteil, das ein am Ende des Führungselements gewinkelt angeordnetes hinteres Griffelement sowie ein am Führungselement schwenkbar gelagertes vorderes Griffelement mit einem Mitnehmerelement, das zur Mitnahme des Kopplungselements ausgeführt ist, aufweist, sowie einer am hinteren Griffelement angeordneten Sicherungseinrichtung, die in einer Sicherungsposition mit einer Formschlusskomponente in einen proximalen Teil einer am Schieberelement angeordneten Führungsnut eingreift. Dabei ist zwischen dem distalen Teil der Führungsnut und dem proximalen Teil ein zur Formschlusskomponente komplementär ausgeführtes Übergangsstück angeordnet.

Die Erfindung umfasst in einer zweiten Alternative weiter ein zerlegbares chirurgisches Schiebeschaftinstrument umfassend einen Funktionsschaft, der ein Führungselement und ein Schieberelement, das an dem Führungselement gleitend gelagert ist und ein Kopplungselement aufweist, sowie eine entlang der Schieberichtung des Schieberelements ausgerichtete hinterschnittene Schiene, die eine Montageöffnung aufweist, und einen Profilsteg mit einem zur Schiene komplementären Profil umfasst, ein Griffteil, das ein am Ende des Führungselements gewinkelt angeordnetes hinteres Griffelement sowie ein am Führungselement schwenkbar gelagertes vorderes Griffelement mit einem Mitnehmerelement, das zur Mitnahme des Kopplungselements ausgeführt ist, aufweist, sowie eines auf einem drehbaren Achsstumpf gelagerten Drehsicherungselements mit einer Verriegelungskomponente, wobei der Achsstumpf in einer am Griffteil drehfest befestigten Buchse gelagert ist, wobei das Drehsicherungselement an dem Funktionsschaft in einer Sicherungsposition formschlüssig angeordnet ist. Die Buchse weist dabei einen Buchsenrand mit einer ersten Formschlussfläche und das Drehsicherungselement eine Ausnehmung mit einer zweiten Formschlussfläche auf, wobei der Buchsenrand in die Ausnehmung eingreift und wobei in einer Öffnungsposition des Drehsicherungselements die zweite Formschlussfläche an der ersten Formschlussfläche angeordnet ist.

Grundgedanke der Erfindung ist, dass mittels einer konstruktiven Maßnahme eine verblüffend effektive Erhöhung der Verriegelungssicherheit erreicht wird.

In der ersten Alternative stellt das komplementär ausgeführte Übergangsstück eine Anschlagsfläche für die Formschlusskomponente der Sicherungseinrichtung bereit. Die Anschlagsfläche bewirkt, dass die Bewegungsfreiheit der Formschlusskomponente in der Sicherungsposition eingeschränkt wird. Durch die einschränkende Bewegungsfreiheit werden ein Verbiegen sowie ein Verschieben der Sicherungseinrichtung effektiv verhindert.

In der zweiten Alternative bewirkt die Erfindung eine Begrenzung der Bewegungsfreiheit des Drehsicherungselements. Mittels einer konstruktiven Maßnahme, wird eine Begrenzung der Drehung des Drehsicherungselements erzielt. Der Rand der Buchse mit der ersten Formschlussfläche der in die Ausnehmung der Drehsicherung eingreift, schlägt bei einer Drehung an die Formschlussfläche der Buchse an, so dass eine weitere Drehung des Drehsicherungselements aufgrund des Formschlusses der Formschlussflächen nicht möglich ist. Damit kann das Drehsicherungselement nicht mehr in der falschen Position eine Verriegelung des Schieberelements bewirken. Dies erhöht die Verriegelungssicherheit des Instruments sowie die Handhabung während der Operation.

Somit wird in beiden alternativen Ausführungsformen der Erfindung durch eine verblüffend einfache konstruktive Maßnahme eine effektive Erhöhung der Verriegelungssicherheit erreicht. Ein Chirurg, der das zerlegbare Schiebeschaftinstrument benutzt, hat nun keine Möglichkeit mehr, versehentlich eine Zerlegung zu bewirken. Damit erreicht die Erfindung eine Erhöhung der Sicherheit in der Handhabung.

Nachfolgend seien einige verwendete Begriffe erklärt:

Unter einer Sicherungsposition wird eine Positionierung der Sicherungseinrichtung bzw. des Drehsicherungselements verstanden, durch die der Öffnungswinkel der Griffelemente des Instruments so begrenzt ist, das eine Zerlegung des Instruments nicht möglich ist. D. h., dass der in die Schiene eingreifende Profilsteg bei maximaler Öffnung der Griffelemente nicht in der Montageöffnung angeordnet ist.

Unter einer Öffnungsposition wird eine Positionierung der Sicherungseinrichtung bzw. des Drehsicherungselements verstanden, durch eine Zerlegung des Instruments möglich ist. Damit ist der Profilsteg bei maximaler Öffnung der Griffelemente in der Montageöffnung angeordnet.

Unter dem proximalen Ende einer Komponente des Schiebeschaftinstruments ist das Ende gemeint, das näher am Griff angeordnet ist als das gegenüberliegende Ende der Komponente. Mit dem distalen Ende einer Komponente wird das Ende bezeichnet, das näher am Maul des Instruments bzw. weiter weg von den Griffen angeordnet ist als das gegenüberliegende Ende der Komponente.

Vorteilhafterweise sind die Formschlusskomponente und das Übergangsstück rechtwinklig ausgestaltet. Durch die rechtwinklige Ausgestaltung wird der Formschluss senkrecht zur Kraft- und Bewegungsrichtung des Schieberelements erreicht. Damit wird eine Verdrehung oder Verbiegung der Sicherungseinrichtung verhindert.

In einer weiteren vorteilhaften Ausführungsform ist das Übergangsstück einstückig mit dem Schieberelement ausgestaltet.

Die Sicherungseinrichtung weist mit Vorteil eine Rückstellfeder auf. Die Feder drückt die Sicherungseinrichtung in die Sicherungsposition. Ohne Krafteinwirkung bleibt die Sicherungseinrichtung damit in der Sicherungsposition. Ein versehentliches Verbleiben in der Öffnungsposition wird durch die Rückstellfeder verhindert.

Vorteilhafterweise weist der Rand der Buchse eine dritte Formschlussfläche und die Ausnehmung des Drehsicherungselements eine vierte Formschlussfläche auf. In einer weiteren Öffnungsposition ist das Drehsicherungselement mit der vierten Formschlussfläche an der dritten Formschlussfläche angeordnet. Dies ermöglicht eine flexiblerere Handhabung des Drehsicherungselements, da es nun ermöglicht ist, die Richtung der Drehung des Drehsicherungselements zum Entriegeln frei zu wählen.

Weiter kann die erste Formschlussfläche vorteilhafterweise spiegelbildlich zur dritten Formschlussfläche und die zweite Formschlussfläche spiegelbildlich zur vierten Formschlussfläche angeordnet sein.

Vorteilhafterweise sind die Formschlussflächen so angeordnet, dass das Drehsicherungselement bei einer Verdrehung aus der Sicherungsposition nur um 90° verdreht werden kann.

In einer weiteren vorteilhaften Ausführungsform ist der Achsstumpf axial verschiebbar. Weiter weist die Buchse in dieser Ausführungsform eine zur Verriegelungskomponente komplementär ausgeführte Ausnehmung auf, wobei die Verriegelungskomponente in der Sicherungsposition des Drehsicherungselements in der Ausnehmung der Buchse angeordnet ist. Damit wird eine Einrastung und somit eine fest definierte Sicherungsposition des Drehsicherungselements erzielt. Weiter ist eine einfache Drehung des Drehsicherungselements nicht mehr möglich. Vielmehr muss das Drehsicherungselement entlang des Achsstumpfs verschoben werden, so dass die Verriegelungskomponente aus der Ausnehmung der Buchse entfernt wird.

Weiter ist der axial verschiebbare Achsstumpf mit der Buchse über eine Rückstellfeder gekoppelt. Die Rückstellfeder zieht den Achsstumpf in Richtung Buchse. Damit kann die eingerastete Drehsicherung sich nicht selbständig aus der Sicherungsposition entriegeln. Zum Entriegeln muss eine Krafteinwirkung durch den Benutzer erfolgen. Dies bewirkt eine weitere Erhöhung der Verriegelungssicherheit.

Die Erfindung wird anhand eines Ausführungsbeispiels gemäß den beigefügten Zeichnungen beschrieben. Es zeigen:

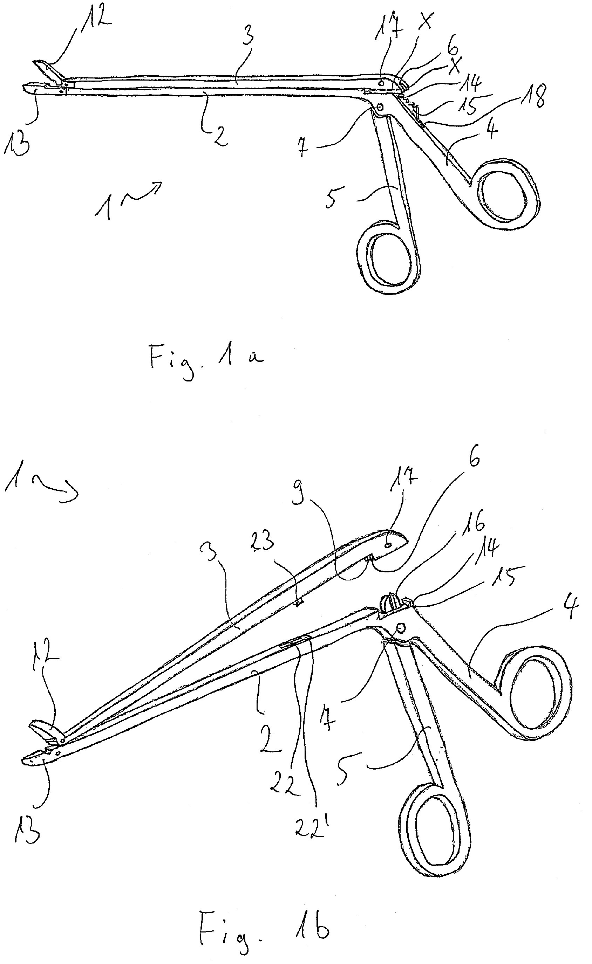

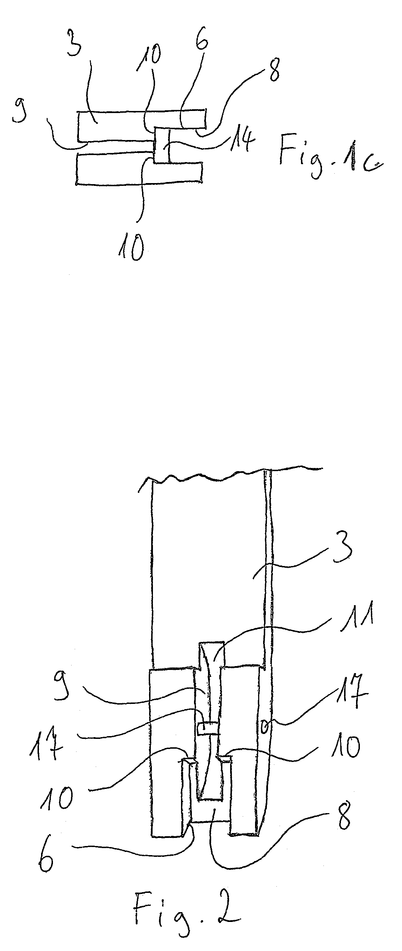

- Fig. 1a, b, c:: Eine schematische Darstellung eines chirurgischen Schiebeschaftinstruments mit einer am hinteren Griffteil angeordneten Sicherungseinrichtung im zusammengebauten Zustand (a), im demontierten Zustand (b), und ein Schnitt durch die Linie X (c);
- Fig. 2:: eine schematische Darstellung des proximalen Teils des Schieberelements von unten;

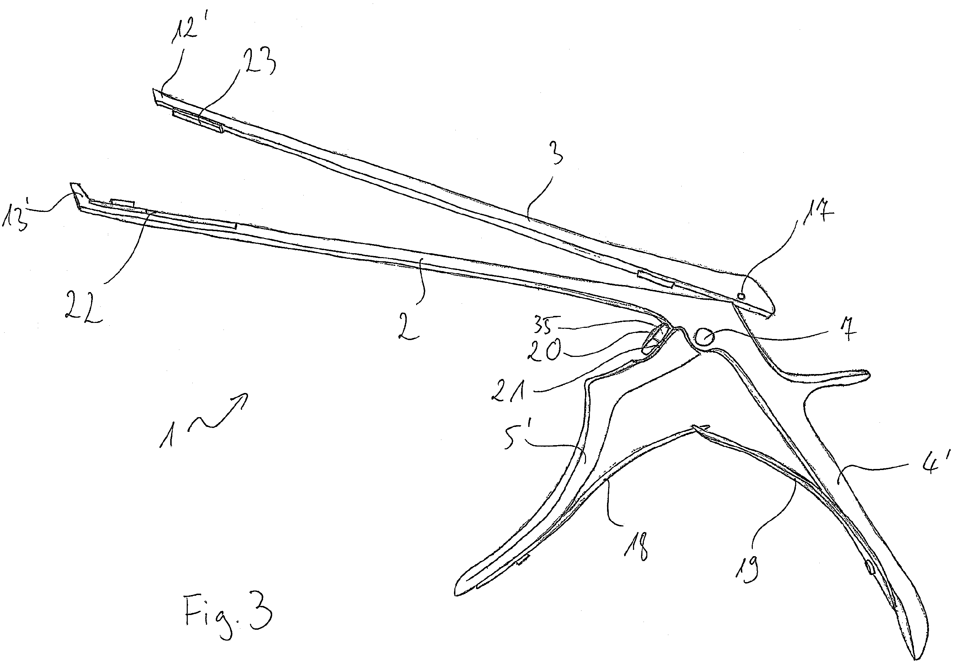

- Fig. 3:: eine schematische Darstellung eines chirurgischen Schiebeschaftinstruments mit Drehsicherung am vorderen Griffteil; und

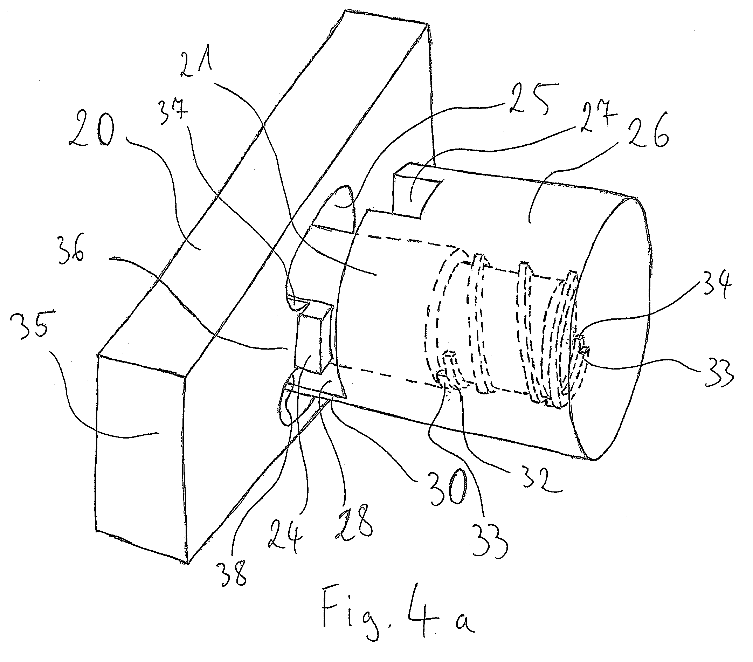

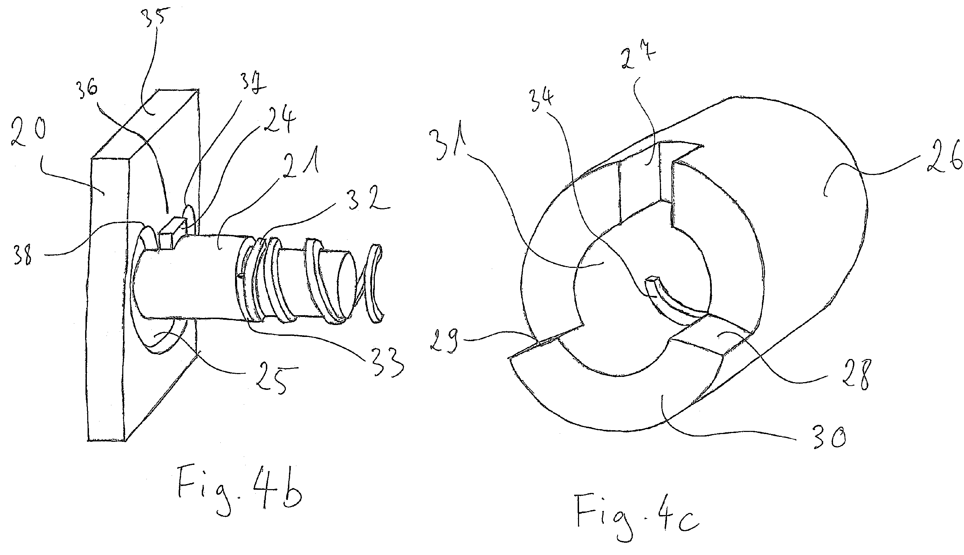

- Fig. 4a, b, c:: eine schematische Darstellung der Drehsicherung mit Buchse (a), der Drehsicherung mit Rückstellfeder (b), und der Buchse (c).

Das zerlegbare chirurgische Schiebeschaftinstrument wird in seiner Gesamtheit mit der Bezugsziffer 1 gekennzeichnet. Es weist einen Funktionsschaft 2, 3 auf, der ein Führungselement 2 und ein Schieberelement 3 aufweist. Weiter weist das Instrument 1 ein Griffteil 4, 5 auf. Das Griffteil 4, 5 umfasst ein vorderes Griffelement 5 und ein hinteres Griffelement 4. Das hintere Griffelement 4 ist abgewinkelt am proximalen Ende des Führungselements 2 angeordnet. Weiter ist das Schieberelement 3 gleitend auf dem Führungselement 2 angeordnet. Das vordere Griffelement 5 ist über ein Scharnierelement 7 am Führungselement 2 schwenkbar gelagert und weist Mitnehmer 16 auf. Am distalen Ende weist das Schiebeschaftinstrument 1 Maulelemente 12, 13 auf. Weiter weist der Funktionsschaft 2, 3 einen Profilsteg 23 und eine zweiteilige hinterschnittene Schiene 22 auf. Sie umfasst an ihrem proximalen Ende eine Montageöffnung 22', durch die der Profilsteg 23 in die Schiene 22 eingeschoben werden kann, wenn das Griffteil 4, 5 in einer Sicherungsposition seinen größten Öffnungswinkel erreicht. Der Profilsteg 23 weist dabei ein Profil auf, das komplementär zum hinterschnittenen Profil der Schiene 22 ausgeführt ist. Ist der Profilsteg 23 in die Schiene 22 eingeschoben, kann das Schieberelement 3 nicht mehr von dem Führungselement 2 angehoben werden.

An dem hinteren Griffelement 4 ist in einer ersten Ausführungsform der Erfindung eine Sicherungseinrichtung 15 angeordnet, die eine Formschlusskomponente 14 umfasst.

Weiter weist das Schieberelement 3 an seinem proximalen Ende eine Führungsnut 6 auf. Die Führungsnut 6 ist in einen proximalen Teil 8 und in einen distalen Teil 9 geteilt. Der proximale Teil 8 ist breiter ausgeführt als der distale Teil 9. Zwischen dem proximalen Teil 8 und dem distalen Teil 9 ist ein Übergangsstück 10 angeordnet, der den breiteren proximalen Teil 8 in den schmaleren distalen Teil 9 überführt. Das Übergangsstück 10 ist komplementär zur Formschlusskomponente 14 ausgestaltet. In dem distalen Teil 9 ist ein Kopplungselement 17 angeordnet, das mit Mitnehmern 16 des vorderen Griffelements 5 gekoppelt wird. Die Mitnehmer 16 greifen dabei in einer bogenförmigen Ausnehmung 11 des distalen Teils 9 der Führungsnut 6 ein. Werden das vordere Griffelement 5 und das hintere Griffelement 4 auseinandergeschwenkt, wird das Schieberelement 3 entlang des Führungselements 2 zum proximalen Ende verschoben. Weiter wird der Profilsteg 23 in Richtung der Montageöffnung 22' geschoben. Ist die Sicherungseinrichtung 15 in einer Sicherungsposition, in der es mit der Formschlusskomponente 14 in den proximalen Teil 8 der Führungsnut 6 eingreift, wird das Übergangsstück 10 an die Formschlusskomponente 14 gedrückt. Durch die zur Formschlusskomponente 14 komplementäre Ausführung des Übergangsstücks 10 umschließt das Übergangsstück 10 in Kombination mit dem proximalen Teil 8 der Führungsnut 6 die Formschlusskomponente 14. Für die Formschlusskomponente 14 bleibt kein Bewegungsspielraum, so dass ein Verbiegen oder Verdrehen der Formschlusskomponente 14 nicht möglich ist. Weiter kann dadurch auch die Sicherungseinrichtung 15 nicht verschoben werden, so dass die Sicherung des Schieberelements 3 gewährleistet bleibt, da der Profilsteg 23 nicht in die Montageöffnung 22' angeordnet werden kann.

Die Sicherungseinrichtung 15 weist eine Rückstellfeder 18 auf, die die Sicherungseinrichtung 15 in die Sicherungsposition drückt. Damit wird die Sicherungseinrichtung 15 automatisch in die Sicherungsposition überführt, sobald keine Kraft auf sie wirkt. Damit wird sichergestellt, dass die Sicherungsposition nur dann verlassen wird, wenn die Sicherungseinrichtung 15 betätigt wird.

In einer zweiten Ausführungsform der Erfindung weist das Schiebeschaftinstrument 1 anstatt der Sicherungseinrichtung 15 ein Drehsicherungselement 20 auf. Das Drehsicherungselement 20 ist auf einem drehbaren Achsstumpf 21 angeordnet, die axial verschiebbar ist. Der Achsstumpf 21 ist in einer Ausnehmung 31 der Buchse 26 angeordnet. Die Buchse 26 ist mit dem Griffteil 5 fest verbunden. Das Drehsicherungselement 20 weist ein Formschlusselement 35 auf, das bei Öffnung der Griffteile 4, 5 formschlüssig an dem Führungselement 2 angeordnet ist. Damit wird ein zu großer Öffnungswinkel zwischen den Griffteilen 4, 5 verhindert. Weiter weist das Drehsicherungselement 20 eine vorragende Verriegelungskomponente 24 auf, das in einer Ausnehmung 27 der Buchse 26 angeordnet ist, wenn das Drehsicherungselement 20 in die Sicherungsposition gedreht ist. Dabei ist die Ausnehmung 27 der Buchse 26 komplementär zur Verriegelungskomponente 24 ausgestaltet. Die Buchse 26 weist weiter einen Rand 30 auf. Der Rand 30 läuft um einen Teil der Ausnehmung 31 um. Der Rand 30 erstreckt sich axial von der Buchse 26 weg. Weiter ist der Rand 30 bogenförmig ausgeführt, d.h., dass er nicht den ganzen Vollkreis umfasst. An den Enden des bogenförmigen Rands 30 ist jeweils eine Formschlussfläche 28, 29 angeordnet. Der Rand 30 greift in eine Ausnehmung 25 des Drehsicherungselements 20 ein. Die Ausnehmung 25 ist bogenförmig um der Achsstumpf 21 angeordnet. Sie beginnt und endet an einem Steg 36, der an der Verriegelungskomponente 24 angeordnet ist. Die am Steg 36 angeordneten Seitenflächen der Ausnehmung 25 weisen Formschlussflächen 37, 38 auf.

Der Achsstumpf 21 ist mit der Buchse 26 über eine Feder 33 gekoppelt. Das eine Ende der Feder 33 wird mittels eines an dem Achsstumpf 21 angeordneten Halteelements 32 mit dem Achsstumpf 21 fest verbunden. Das andere Ende der Feder 33 ist mit einem weiteren Halteelement 34 in der Ausnehmung 31 mit der Buchse 26 fest verbunden. Die Feder 33 zieht den Achsstumpf 21 in die Buchse 26 hinein.

Um das Drehsicherungselement 20 zu verdrehen, muss es zunächst in axialer Richtung aus der Buchse 26 herausgezogen werden. Dabei muss die Federkraft der Feder 33 überwunden werden und es wird auch die Verriegelungskomponente 24 aus der Ausnehmung 27 entfernt. Durch eine Drehung wird das Formschlusselement 35 von dem Führungselement 2 weggedreht. Die Drehung des Drehsicherungselements 20 wird durch den Kontakt der Formschlussfläche 28 des Randes 30 und der Formschlussfläche 38 der Ausnehmung 25 bzw. der Formschlussfläche 29 des Rands 30 und der Formschlussfläche 37 der Ausnehmung 25 blockiert. Eine weitere Verdrehung ist nicht möglich. Der Rand 31 umschließt dabei einen Winkel, der so ausgeführt ist, dass das Drehsicherungselement 20 nur um 90° aus der Sicherungsposition verdreht werden kann. Damit ist es nicht möglich, das Formschlusselement 35 in eine vom Führungselement 2 weg weisende Richtung zu drehen. Die Begrenzung des Öffnungswinkels des Griffteils 4, 5 ist damit nur durch das Eindrehen der Formschlusskomponente 35 zum Führungselement 2 möglich.

Die Feder 33 bewirkt, dass das Drehsicherungselement 20 nur dann aus der Sicherungsposition gezogen werden kann, wenn eine Krafteinwirkung von außen erfolgt. Ist keine Krafteinwirkung von außen vorhanden, verbleibt das Drehsicherungselement 20 in der Sicherungsposition. Damit wird die Sicherheit der Verriegelung erhöht.

In beiden Ausführungsformen können die Griffelemente 4, 5 wie die Griffe einer Schere ausgeführt sein oder wie die Griffelemente 4', 5' einer Zange. Weiter können Federelemente 18, 19 an den Griffteilen 4', 5' vorgesehen sein, die eine automatische Öffnung der Griffteile 4', 5' nach einer Betätigung bewirken. Es können weiter verschiedene Maulelemente 12, 13 am Schiebeschaftinstrument 1 vorgesehen werden. So können die Maulelemente 12, 13 aus Klingen bestehen. In einer anderen Ausführungsform können die Maulelemente als Stanzen 12', 13' ausgeführt sein.

## Patentansprüche

1. Zerlegbares chirurgisches Schiebeschaftinstrument umfassend einen Funktionsschaft (2, 3), der ein Führungselement (2) und ein Schieberelement (3), das an dem Führungselement (2) gleitend gelagert ist und ein Kopplungselement (17) aufweist, sowie eine entlang der Schieberichtung des Schieberelements (3) ausgerichtete hinterschnittene Schiene (22), die an ihrem proximalen Ende eine Montageöffnung (22') aufweist, und einen Profilsteg (23) mit einem zur Schiene (22) komplementären Profil umfasst, ein Griffteil (4, 5), das ein am Ende des Führungselements (2) gewinkelt angeordnetes hinteres Griffelement (4) sowie ein am Führungselement (2) schwenkbar gelagertes vorderes Griffelement (5) mit einem Mitnehmerelement (16), das zur Mitnahme des Kopplungselements (17) ausgeführt ist, aufweist, sowie einer am hinteren Griffelement (4) angeordneten Sicherungseinrichtung (15),
**dadurch gekennzeichnet, dass**
die Sicherungseinrichtung in einer Sicherungsposition mit einer Formschlusskomponente (14) in einen proximalen Teil (8) einer am Schieberelement (3) angeordneten Führungsnut (6) eingreift,
wobei zwischen einem distalen Teil (9) der Führungsnut (6) und dem proximalen Teil (8) ein zur Formschlusskomponente (14) komplementär ausgeführtes Übergangsstück (10) angeordnet ist.

2. Schiebeschaftinstrument nach Anspruch 1,
**dadurch gekennzeichnet, dass**
das Übergangsstück (10) rechtwinklig ausgeführt ist.

3. Schiebeschaftinstrument nach den Ansprüchen 1 oder 2,
**dadurch gekennzeichnet, dass**
das Übergangsstück (10) einstückig mit dem Schieberelement (3) ist.

4. Schiebeschaftinstrument nach den Ansprüchen 1 bis 3,
**dadurch gekennzeichnet, dass**
die Sicherungseinrichtung (15) eine Rückstellfeder (18) aufweist.

5. Zerlegbares chirurgisches Schiebeschaftinstrument umfassend einen Funktionsschaft (2, 3), der ein Führungselement (2) und ein Schieberelement (3), das an dem Führungselement (2) gleitend gelagert ist und ein Kopplungselement (17) aufweist, sowie eine entlang der Schieberichtung des Schieberelements (3) ausgerichtete hinterschnittene Schiene (22), die an ihrem proximalen Ende eine Montageöffnung (22') aufweist, und einen Profilsteg (23) mit einem zur Schiene (22) komplementären Profil umfasst, ein Griffteil (4, 5), das ein am Ende des Führungselements (2) gewinkelt angeordnetes hinteres Griffelement (4) sowie ein am Führungselement (2) schwenkbar gelagertes vorderes Griffelement (5) mit einem Mitnehmerelement (16), das zur Mitnahme des Kopplungselements (17) ausgeführt ist, aufweist, sowie eines auf einem drehbaren Achsstumpf (21) gelagerten Drehsicherungselements (20) mit einer Verriegelungskomponente (24), wobei der Achsstumpf (21) in einer am Griffteil (4, 5) drehfest befestigten Buchse (26) gelagert ist, wobei das Drehsicherungselement (20) an dem Funktionsschaft (2, 3) in einer Sicherungsposition formschlüssig angeordnet ist,
**dadurch gekennzeichnet, dass**
die Buchse (26) einen Buchsenrand (30) mit einer ersten Formschlussfläche (28) und das Drehsicherungselement (20) eine Ausnehmung (25) mit einer zweiten Formschlussfläche (38) aufweist, wobei der Buchsenrand (30) in die Ausnehmung (25) eingreift und wobei in einer Öffnungsposition des Drehsicherungselements (20) die zweite Formschlussfläche (38) formschlüssig an der ersten Formschlussfläche (28) angeordnet ist.

6. Schiebeschaftinstrument nach Anspruch 5,
**dadurch gekennzeichnet, dass**
der Buchsenrand (30) eine dritte Formschlussfläche (29) und die Ausnehmung (25) eine vierte Formschlussfläche (37) aufweist, wobei in einer zweiten Öffnungsposition des Drehsicherungselements (20) die vierte Formschlussfläche (37) formschlüssig an der dritten Formschlussfläche (29) angeordnet ist.

7. Schiebeschaftinstrument nach Anspruch 6,
**dadurch gekennzeichnet, dass**
die erste Formschlussfläche (28) spiegelbildlich zur dritten Formschlussfläche (29) und die zweite Formschlussfläche (38) spiegelbildlich zur vierten Formschlussfläche (37) angeordnet ist.

8. Schiebeschaftinstrument nach den Ansprüchen 5 bis 7,
**dadurch gekennzeichnet, dass**
das der Buchsenrand (30) so ausgeführt ist, dass in jeder Öffnungsposition das Drehsicherungselement (20) um höchstens 90° aus der Sicherungsposition verdreht ist.

9. Schiebeschaftinstrument nach den Ansprüchen 5 bis 8,
**dadurch gekennzeichnet, dass**
der Achsstumpf (21) axial verschiebbar ist und die Buchse (26) eine zur Verriegelungskomponente (24) komplementär ausgestaltete Ausnehmung (27) aufweist, wobei das Drehsicherungselement (20) in eine Sicherungsposition mit der Verriegelungskomponente (24) in der Ausnehmung (27) der Buchse (26) angeordnet ist.

10. Schiebeschaftinstrument nach Anspruch 9,
**dadurch gekennzeichnet, dass**
der Achsstumpf (21) mittels einer Feder (33) an die Buchse (26) gekoppelt ist.

## Claims

1. Dismantlable surgical sliding shaft instrument, comprising a function shaft (2, 3), which has a guide element (2) and a slide element (3) mounted slidably on the guide element (2) and has a coupling element (17), and comprising an undercut rail (22), which is oriented along the sliding direction of the slide element (3) and has a mounting opening (22') at its proximal end, and a profile web (23) with a profile that matches the rail (22), a grip part (4, 5), which has a rear grip element (4) arranged at an angle at the end of the guide element (2), and a front grip element (5) mounted pivotably on the guide element (2), which front grip element (5) has a carrier element (16) designed to carry along the coupling element (17), and a safety device (15) arranged on the rear grip element (4), **characterized in that** the safety device engages with a form-fit component (14) in a proximal part (8) of a guide groove (6), arranged on the slide element (3), in a safety position, wherein a transition piece (10) matching the form-fit component (14) is arranged between a distal part (9) of the guide groove (6) and the proximal part (8).

2. Sliding shaft instrument according to Claim 1, **characterized in that** the transition piece (10) is configured at a right angle.

3. Sliding shaft instrument according to Claim 1 or 2, **characterized in that** the transition piece (10) is integral with the slide element (3).

4. Sliding shaft instrument according to Claims 1 to 3, **characterized in that** the safety device (15) has a restoring spring (18).

5. Dismantlable surgical sliding shaft instrument, comprising a function shaft (2, 3), which has a guide element (2) and a slide element (3) mounted slidably on the guide element (2) and has a coupling element (17), and comprising an undercut rail (22), which is oriented along the sliding direction of the slide element (3) and has a mounting opening (22') at its proximal end, and a profile web (23) with a profile that matches the rail (22), a grip part (4, 5), which has a rear grip element (4) arranged at an angle at the end of the guide element (2), and a front grip element (5) mounted pivotably on the guide element (2), which front grip element (5) has a carrier element (16) designed to carry along the coupling element (17), and a rotation safety element (20), which is mounted on a rotatable pin stub (21) and has a locking component (24), wherein the pin stub (21) is mounted in a bushing (26) secured in a rotationally fixed manner on the grip part (4, 5), wherein the rotation safety element (20) is arranged with a form fit on the function shaft (2, 3) in a safety position, **characterized in that** the bushing (26) has a bushing edge (30) with a first form-fit surface (28), and the rotation safety element (20) has a recess (25) with a second form-fit surface (38), wherein the bushing edge (30) engages in the recess (25), and wherein the second form-fit surface (38) is arranged with a form fit on the first form-fit surface (28) in an opening position of the rotation safety element (20).

6. Sliding shaft instrument according to Claim 5, **characterized in that** the bushing edge (30) has a third form-fit surface (29), and the recess (25) has a fourth form-fit surface (37), wherein the fourth form-fit surface (37) is arranged with a form fit on the third form-fit surface (29) in a second opening position of the rotation safety element (20).

7. Sliding shaft instrument according to Claim 6, **characterized in that** the first form-fit surface (28) is arranged mirror-symmetrically with respect to the third form-fit surface (29), and the second form-fit surface (38) is arranged mirror-symmetrically with respect to the fourth form-fit surface (37).

8. Sliding shaft instrument according to Claims 5 to 7, **characterized in that** the bushing edge (30) is configured such that, in each opening position, the rotation safety element (20) is rotated through a maximum of 90° from the safety position.

9. Sliding shaft instrument according to Claims 5 to 8, **characterized in that** the pin stub (21) is axially displaceable, and the bushing (26) has a recess (27) matching the locking component (24), wherein the rotation safety element (20) is arranged in a safety position with the locking component (24) in the recess (27) of the bushing (26).

10. Sliding shaft instrument according to Claim 9, **characterized in that** the pin stub (21) is coupled to the bushing (26) by means of a spring (33).

## Revendications

1. Instrument chirurgical à tige coulissante démontable comprenant une tige fonctionnelle (2, 3), qui présente un élément de guidage (2) et un élément de coulisseau (3) qui est supporté de manière à pouvoir coulisser sur l'élément de guidage (2) et un élément d'accouplement (17), ainsi qu'un rail (22) en contre-dépouille réalisé le long de la direction de coulissement de l'élément de coulisseau (3) qui présente au niveau de son extrémité proximale une ouverture de montage (22'), et qui comprend une nervure profilée (23) avec un profil complémentaire du rail (22), une partie de préhension (4, 5) qui présente un élément de préhension arrière (4) disposé de manière coudée à l'extrémité de l'élément de guidage (2) ainsi qu'un élément de préhension avant (5) supporté de manière à pouvoir pivoter sur l'élément de guidage (2) avec un élément d'entraînement (16) qui est réalisé pour entraîner l'élément d'accouplement (17), ainsi qu'un dispositif de fixation (15) disposé au niveau de l'élément de préhension arrière (4),
**caractérisé en ce que**
le dispositif de fixation s'engage dans une position de fixation avec un composant d'engagement par correspondance de formes (14) dans une partie proximale (8) d'une rainure de guidage (6) disposée sur l'élément coulissant (3),
une pièce de transition (10) réalisée de manière complémentaire au composant d'engagement par correspondance de formes (14) étant disposée entre une partie distale (9) de la rainure de guidage (6) et la partie proximale (8).

2. Instrument à tige coulissante selon la revendication 1, **caractérisé en ce que** la pièce de transition (10) est réalisée à angle droit.

3. Instrument à tige coulissante selon les revendications 1 ou 2, **caractérisé en ce que** la pièce de transition (10) est réalisée d'une seule pièce avec l'élément de coulisseau (3).

4. Instrument à tige coulissante selon les revendications 1 à 3, **caractérisé en ce que** le dispositif de fixation (15) présente un ressort de rappel (18).

5. Instrument chirurgical à tige coulissante démontable comprenant une tige fonctionnelle (2, 3), qui présente un élément de guidage (2) et un élément de coulisseau (3) qui est supporté de manière à pouvoir coulisser sur l'élément de guidage (2) et un élément d'accouplement (17), ainsi qu'un rail (22) en contre-dépouille réalisé le long de la direction de coulissement de l'élément de coulisseau (3) qui présente au niveau de son extrémité proximale une ouverture de montage (22'), et qui comprend une nervure profilée (23) avec un profil complémentaire du rail (22), une partie de préhension (4, 5) qui présente un élément de préhension arrière (4) disposé de manière coudée à l'extrémité de l'élément de guidage (2) ainsi qu'un élément de préhension avant (5) supporté de manière à pouvoir pivoter sur l'élément de guidage (2) avec un élément d'entraînement (16) qui est réalisé pour entraîner l'élément d'accouplement (17), ainsi qu'un élément de fixation en rotation (20) supporté sur un bout d'axe rotatif (21) avec un composant de verrouillage (24), le bout d'axe (21) étant supporté dans une douille (26) fixée de manière solidaire en rotation à la partie de préhension (4, 5), l'élément de fixation en rotation (20) étant disposé par engagement par correspondance de formes sur la tige fonctionnelle (2, 3) dans une position de fixation, **caractérisé en ce que** la douille (26) présente un bord de douille (30) avec une première surface d'engagement par correspondance de formes (28) et l'élément de fixation en rotation (20) présente un évidement (25) avec une deuxième surface d'engagement par correspondance de formes (38), le bord de douille (30) s'engageant dans l'évidement (25) et, dans une position d'ouverture de l'élément de fixation en rotation (20), la deuxième surface d'engagement par correspondance de formes (38) étant disposée par engagement par correspondance de formes sur la première surface d'engagement par correspondance de formes (28).

6. Instrument à tige coulissante selon la revendication 5, **caractérisé en ce que** le bord de douille (30) présente une troisième surface d'engagement par correspondance de formes (29) et l'évidement (25) présente une quatrième surface d'engagement par correspondance de formes (37), dans une deuxième position d'ouverture de l'élément de fixation en rotation (20), la quatrième surface d'engagement par correspondance de formes (37) étant disposée par engagement par correspondance de formes sur la troisième surface d'engagement par correspondance de formes (29).

7. Instrument à tige coulissante selon la revendication 6, **caractérisé en ce que** la première surface d'engagement par correspondance de formes (28) est disposée avec une symétrie spéculaire par rapport à la troisième surface d'engagement par correspondance de formes (29) et la deuxième surface d'engagement par correspondance de formes (38) est disposée avec une symétrie spéculaire par rapport à la quatrième surface d'engagement par correspondance de formes (37).

8. Instrument à tige coulissante selon les revendications 5 à 7, **caractérisé en ce que** le bord de douille (30) est réalisé de telle sorte que dans chaque position d'ouverture, l'élément de fixation en rotation (20) soit tourné de 90° au maximum à partir de la position de fixation.

9. Instrument à tige coulissante selon les revendications 5 à 8, **caractérisé en ce que** le bout d'axe (21) est déplaçable axialement et la douille (26) présente un évidement (27) configuré de manière complémentaire au composant de verrouillage (24), l'élément de fixation en rotation (20) étant disposé dans une position de fixation avec le composant de verrouillage (24) dans l'évidement (27) de la douille (26).

10. Instrument à tige coulissante selon la revendication 9, **caractérisé en ce que** le bout d'axe (21) est accouplé au moyen d'un ressort (33) à la douille (26).
